# EUROPÄISCHE PATENTSCHRIFT

(11) **EP 3 790 927 B1**
(45) Veröffentlichungstag und Bekanntmachung des Hinweises auf die Patenterteilung: **23.02.2022**
(21) Anmeldenummer: 19774057.4
(22) Anmeldetag: 04.09.2019
(51) Int. Cl.: C08J 11/24, C08L 67/02

(54) **VERFAHREN, VORRICHTUNG UND VERWENDUNG ZUR WIEDERAUFARBEITUNG VON IM WESENTLICHEN POLYALKYLENTEREPHTHALAT**
METHOD, DEVICE AND USE FOR REPROCESSING SUBSTANTIALLY POLYALKYLENE TEREPHTHALATE
PROCÉDÉ, DISPOSITIF ET UTILISATION ESSENTIELLEMENT DE POLYALKYLÈNETÉRÉPHTHALATE À DES FINS DE RETRAITEMENT

(30) Priorität: 12.09.2018 DE 102018122210
(43) Veröffentlichungstag der Anmeldung: 17.03.2021
(73) Patentinhaber: RITTEC Umwelttechnik GmbH, 21335 Lüneburg (DE)
(72) Erfinder: EICHERT, Carsten, 21391 Reppenstedt-Dachtmissen (DE); BREPOHL, Esther, 38116 Braunschweig (DE); SCHOLL, Stephan, 38116 Braunschweig (DE); BIERMANN, Lars, 31177 Harsum (DE)
(74) Vertreter: VKK Patentanwälte PartG mbB
(86) Internationale Anmeldenummer: PCT/EP2019/073598
(87) Internationale Veröffentlichungsnummer: WO 2020/053051

(56) Entgegenhaltungen:
- EP-A1- 1 710 226
- WO-A1-94/10121
- WO-A1-2015/190941
- DE-A1- 10 058 773
- JP-A- H09 286 744
- JP-A- H11 302 208
- US-A- 3 544 622
- DATABASE WPI Week 200756 Thomson Scientific, London, GB; AN 2007-577497 XP002796867, & JP 2007 153942 A (TORAY IND INC) 21. Juni 2007 (2007-06-21)

## Beschreibung

Die vorliegende Erfindung betrifft ein Verfahren zur Wiederaufarbeitung von im wesentlichen Polyalkylenterephthalat, insbesondere Polyalkylenterephthalat und/oder Polyalkylenterephthalat, enthaltenden Abfällen in einem kontinuierlichen Prozess mittels einer Depolymerisation, wobei zur Herstellung eines Reaktionsgemisches den Abfällen ein, vorzugsweise festes, Alkali- und/oder Erdalkalihydroxid, insbesondere Natriumhydroxid, beigefügt wird.

Die vorliegende Erfindung betrifft gleichermaßen eine Vorrichtung zur Durchführung eines derartigen Verfahrens.

Schließlich betrifft die vorliegende Erfindung die Verwendung einer derartigen Vorrichtung zur Durchführung eines derartigen Verfahrens.

Die Erfindung betrifft insbesondere ein kontinuierliches Verfahren zum Recycling von Polyalkylenterephthalat enthaltenden Abfällen, bei dem die Abfälle geeignet vorbereitet mit Alkalimetall- oder Erdalkalimetallhydroxid in einem Extruder oder Knetreaktor vermischt und erwärmt werden.

Der Hauptvorteil des erfindungsgemäßen Verfahrens besteht darin, dass es die kontinuierliche Aufarbeitung von Polyalkylenterephthalat enthaltenden Abfällen sowie mehrschichtigen Polyalkylenterephthalat enthaltenden Abfällen erlaubt. Die kontinuierliche Aufarbeitung ermöglicht die kontinuierliche Gewinnung eines Alkalimetall- oder Erdalkalimetallterephthalat enthaltenden Wertstoffstroms sowie die Abtrennung und Gewinnung des gebildeten und eingesetzten Alkylenglykols. Der Alkalimetall- oder Erdalkalimetallterephthalat enthaltenden Wertstoffstrom kann anschließend in einem geeigneten Lösungsmittel, zum Beispiel Wasser, gelöst, gereinigt und gegebenenfalls in Terephthalsäure (TPA) oder einen Terephthalsäureester überführt werden.

Zur Herstellung von TPA oder einem Zwischenprodukt der TPA aus Polyalkylenterephthalat und insbesondere Polyethylenterephthalat (PET) in Form von Abfällen sind verschiedene Verfahren bekannt. Diese behandeln jedoch keine mehrschichtigen PET-Abfälle und sind weder effizient noch wirtschaftlich von Vorteil. Im Folgenden werden diese kurz vorgestellt.

In dem US-Patent 4542239 wird ein Verfahren zur Gewinnung von TPA aus PET-Abfällen mit wässrigem Ammoniumhydroxid beschrieben. Für die Durchführung des Verfahrens sind sowohl ein erhöhter Druck als auch eine erhöhte Temperatur notwendig. Zudem sind umfangreiche Sicherheitsanforderungen beim Einsatz von Ammoniumhydroxid zu erfüllen.

In den US-Patenten 3120561 und 4578502 wird die Depolymerisation von PET in Anwesenheit von Wasser oder Methanol durch Hydrolyse realisiert. Dabei werden über mehrere Stunden ebenso eine hohe Temperatur und ein hoher Druck benötigt, um anschließend durch Kühlung TPA zu gewinnen.

In dem US-Patent 4355175 erfolgt die Hydrolyse der PET-Abfälle mit verdünnter Schwefelsäure. Anschließend wird die Lösung mit einer alkalischen Lösung versetzt, um ausgefällte Verunreinigungen durch Filtration abtrennen zu können. Durch Zugabe von Schwefelsäure wird TPA gewonnen.

In dem US-Patent 3952053 wird ein Verfahren zur Behandlung von Polyester-Produktionsabfällen beschrieben. Dabei erfolgt zunächst die Zugabe von Schwefelsäure, um anschließend Farbstoffe und Additive entfernen zu können. Dieses gereinigte Zwischenprodukt wird mit Natronlauge versetzt, sodass TPA ausfällt. Das enthaltene Monoethylenglykol (MEG) wird durch Destillation wiedergewonnen.

In dem deutschen Patent 69714614 wird eine wässrige, leicht alkalische Lösung bei erhöhter Temperatur und erhöhtem Druck zur Depolymerisation von PET verwendet. Für die alkalische Lösung werden Reagenzien aus der Gruppe der Bicarbonate von Ammoniak und Alkalimetallen, Ammoniumcarbamat und Harnstoff eingesetzt. Das freigesetzte Kohlenstoffdioxid wird recycelt.

In dem deutschen Patent 69522479 erfolgt die Depolymerisation mit einem Lösungsmittel (beispielsweise Wasser) und einem Benetzungsmittel in Gegenwart von einem Alkalimetall- oder Erdalkalimetallhydroxid bei erhöhter Temperatur und erhöhtem Druck. Nach der Filtration des gelösten Alkalimetall- oder Erdalkalimetallterephthalats und der Fällung der TPA mittels einer Säure wird ein Kristallisationsverfahren durchgeführt, um die TPA-Partikel zu vergrößern.

In dem US-Patent 5395858 werden PET-Abfällen und silberhaltige PET-Abfällen (Foto- und Röntgenfilme) in einer Natriumhydroxidlösung depolymerisiert. Durch die anschließende Verdampfung des Lösungsmittels bleibt Dinatriumterephthalat zurück, das in Wasser gelöst und mit einer Säure zur TPA umgesetzt wird.

In dem US-Patent 3544622 wird die Verseifung von PET mit Natronlauge und Ethylenglykol bei Atmosphärendruck und mindestens 150°C beschrieben. Diese Depolymerisation erfolgt absatzweise in einem Rührkessel bei gleichzeitiger Verdampfung des Ethylenglykols. Das dabei entstehende Dinatriumterephthalat wird ebenfalls mittels einer Säure zu TPA umgesetzt.

In dem US-Patent 6720448 B2 wird PET bei erhöhter Temperatur wasserfrei, beispielsweise in Ethylenglykol, mit einem Salz, dass eine schwächere Säure als TPA ist, umgesetzt. Dabei werden verschiedene Basen und deren Mischungen eingesetzt. Anschließend wird das Zwischenprodukt in Wasser gelöst, gefiltert und die TPA durch Zugabe einer starken Säure erhalten.

In dem US-Patent 2017/0152203 A1 wird die Depolymerisation von PET bei Temperaturen zwischen 20 und 60°C in Dichlormethan/Methanolmischungen beschrieben. Daneben wird noch der Einsatz diverser weiterer Lösungsmittel genannt, um anschließend TPA und Ethylenglykol wiederzugewinnen. Zudem wird das Quellen des Polymers durch beispielsweise unpolare Lösungsmittel beschrieben. Die Durchführung der Depolymerisation erfolgt absatzweise, teilweise über viele Stunden.

In dem deutschen Patent 69316545 T2 wird ein Verfahren zur Depolymerisation von nicht-beschichtetem PET mittels eines Alkalimetall- oder Erdalkalimetallhydroxids in einem Knetextruder beschrieben. Dabei wird kein Lösungsmittel hinzugegeben. Das Gemisch wird anschließend im Knetextruder erwärmt und wenigstens teilweise aufgeschmolzen. Im Anschluss wird das erhaltene Alkalimetall- oder Erdalkalimetallterephthalat in Wasser gelöst und gefiltert, um mit Hilfe von Schwefelsäure die TPA zu gewinnen.

Bergmann et al. beschreiben in "On-Line Monitoring of Molecular Weight Using NIR Spectroscopy in Reactive Extrusion Process" in Macromolecular Symposia 2013 die Glykolyse von PET in einem Extruder bei einer Temperatur von 320°C. Dabei wird Ethylenglykol eingesetzt, um das PET zu depolymerisieren. Es wird jedoch keine TPA gewonnen.

Bei den zuvor beschriebenen Verfahren erfolgt der Umsatz des PETs überwiegend bei hohen Temperaturen und hohen Drücken. Dies hat den Nachteil, dass der apparative und energetische Aufwand sehr hoch ist und somit die Wirtschaftlichkeit der Verfahren vermindert ist. Ebenso werden die meisten der beschriebenen Verfahren nur absatzweise durchgeführt. Angesichts der hohen Temperaturen und hohen Drücke sind durch die im Stand der Technik vorgesehene absatzweise Bearbeitung jedoch die Aufwendungen für das Aufheizen und den Druckaufbau mit Nachteil erheblich.

Insbesondere das Recycling für mehrschichtige Verbundmaterialien auf Polymerbasis stellte auf Grund der stofflichen Verbindung unterschiedlicher Materialien mit dem Polyalkylenterephthalat, sehr hohe Verfahrensanforderungen. Derartige Verbundsysteme werden insbesondere als Mehrschichtverpackungen im Lebensmittelbereich eingesetzt, um einerseits mechanisch stabile Verpackungen und andererseits die erforderlichen Schutzfunktionen für das aufzunehmende Lebensmittel aufzuweisen. Um diese Anforderungen an eine Verpackung zu erfüllen, wird auf die Verwendung von Zwei- oder Mehrschichtverpackungen zurückgegriffen. Diese Verpackungen bestehen aus mehreren Schichten aus verschiedenen Polymeren oder Materialien und/oder anorganischen Beschichtungen, die in der Regel jeweils mindestens eine Funktion haben. Beispielsweise wird Ethylen-Vinylacetat Copolymer in Lebensmittelverpackungen als Sauerstoffbarriere eingesetzt. Der Aufbau der Multilayerverpackungen (Mehrschichtsystemverpackung) wird beispielsweise in den Patentschriften US9475251B2, US6610392B1 und EP1036813A1 beschrieben. Eine weit verbreitete Lebensmittelverpackung besteht beispielsweise aus einer PET-Schale, die mit einer dünnen Lage aus Polyethylen (PE) oder Polyamid (PA) beschichtet ist. Es besteht bei diesen und den anderen Mehrschichtverpackungen ein fester Materialverbund der verschiedenen Polymere oder Materialien. Nach dem heutigen Stand der Technik sind Mehrschichtmaterialien kaum oder nur schwer wiederverwertbar. In dem Patent WO2003104315A1 ist ein Ansatz beschrieben, welches ein Verfahren für die Trennung von Mehrschichtsystemen beschreibt, bei dem es zu keiner Depolymerisation, Auflösung oder Oxidation des eingesetzten Materials kommt. Allerdings verwendet das Verfahren umweltgefährdende Lösungsmittel und wurde nach dem Kenntnisstand der Autoren noch nicht wirtschaftlich umgesetzt. Mit dem Ansatz, der im Patent WO2003070376A1 beschrieben wird, können beschichtete Kunststoff-Formkörper aus einem PET-Formkörper, einer Barriereschicht aus Polyvinylalkohol und einer Deckschicht durch den Einsatz von Wasser getrennt werden. Hier wird die Barriere- und Zwischenschicht aus Polyvinylalkohol aufgelöst und es kann dadurch der Formkörper von der Deckschicht getrennt werden. Das Verfahren ist dadurch mit Nachteil auf sehr spezifische Dreischichtsysteme begrenzt.. Aufgrund der Schwierigkeit, die verschiedenen Schichten voneinander zu trennen, werden derartige Mehrschichtsysteme beziehungsweise Multilayermaterialien nach deren Verwendung nach dem heutigen Stand der Technik großtechnisch lediglich thermisch verwertet oder in Deponien verfüllt. Sowohl bei der thermischen Verwertung als auch bei der Verfüllung des Abfalls in Deponien geht der Werkstoff aus dem Stoffkreislauf verloren. Eine Übersicht über die verschiedenen Verpackungen, die in der Lebensmittelindustrie verwendet werden, liefern Kaiser et al. "Recycling of Polymer-Based Multilayer Packaging: A Review" in Recycling 2018.

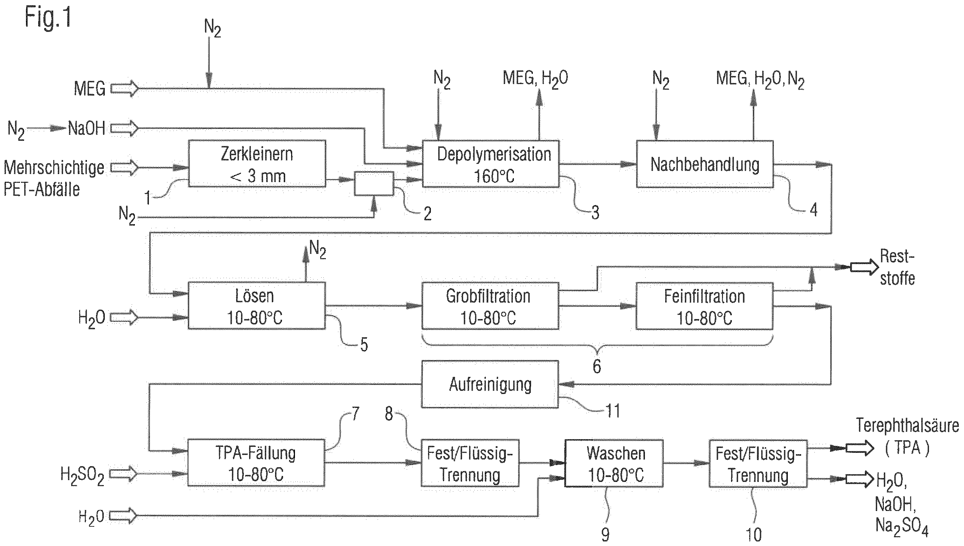

In dem deutschen patent DE 100 58 773 wird ein Verfahren zur Rückgewinnung von Terephthalsäure aus dem pulverisierten Produkt von verbrauchtem Polyethylenterephthalat und System für die Verwendung in diesem Verfahren beschrieben. DE 100 58 773 offenbart zwar in den Figuren 1 und 2 einen Extruder 1 mit einer einfachen Förderschnecke 2. Jedoch ist weder in den Figuren 1 und 2 noch in einer der weiteren Figuren ein Doppelschneckenextruder offenbart.

Vor dem geschilderten Hintergrund liegt der vorliegenden Erfindung die Aufgabe zu Grunde, ein Verfahren, eine Vorrichtung sowie eine Verwendung der eingangs genannten Art zur Wiederaufarbeitung von im wesentlichen Polyalkylenterephthalat, insbesondere Polyethylenterephthalat und/oder Polybuthylenterephthalat, enthaltenden Abfällen in einem kontinuierlichen Prozess mittels einer Depolymerisation anzugeben, welches geeignet ist, mit einem hohen Durchsatz auch Mehrschichtsysteme und farbige Materialien nahezu vollständig chemisch in die Ausgangsstoffe mit hoher Qualität zu recyceln, um aus den Recyclingprodukten uneingeschränkt neue Polyalkylenterephthalatprodukte herstellen zu können.

Erfindungsgemäß wird die Aufgabe gelöst durch die Merkmalskombination der unabhängigen Patentansprüche.

Insbesondere wird die auf ein Verfahren gerichtete Aufgabe bei einem Verfahren der eingangs genannten Art dadurch gelöst, dass dem Reaktionsgemisch als Edukt zusätzlich ein Alkylenglykol beigefügt wird, wobei das Alkylenglykol ein als Produkt der angestrebten Depolymerisation herstellbares Alkylenglykol, insbesondere MEG, ist und wobei dem Reaktionsgemisch keine weiteren reaktiven Bestandteile beigefügt werden. Es hat sich im Rahmen der Erfindung gezeigt, dass die Hinzufügung als Edukt eines bei der späteren Depolymerisation entstehenden Alkylenglykols eine optimierte Verfahrensführung hinsichtlich der Recyclingraten und der Recyclingqualität ermöglicht. Insbesondere wird erfindungsgemäß bei der Wiederaufarbeitung von PET-Abfällen neben beispielsweise Natriumhydroxid auch MEG beigefügt.

In weiterer vorteilhafter Ausgestaltung des erfindungsgemäßen Verfahrens werden die Abfälle vorder Herstellung des Reaktionsgemisches, vorzugsweise auf eine Größe von höchstens 3 mm, zerkleinert. Durch diese Maßnahme wird schon bei Herstellung des Reaktionsgemisches, also noch vor der Durchführung der eigentlichen Depolymerisation, erreicht, dass die Abfälle, insbesondere Mehrschichtsysteme, mechanisch zermahlen und aufgebrochen werden um eine möglichst große Oberfläche für die Verseifungsreaktion bereitzustellen. Durch die mechanische Zerkleinerung werden der Materialverbund zwischen den verschiedenen Schichten sowie die Schichten selbst geschädigt, sodass erfindungsgemäß eine Reaktion von allen bzw. von verschiedenen Seiten an dem Abfall, insbesondere PET, stattfinden kann.

In bevorzugter Ausgestaltung des erfindungsgemäßen Verfahrens wird das Alkylenglykol mit einem Massenstrom beigefügt, welcher derart gewählt wird, dass das Massenstromverhältnis von den Abfällen zu Alkylenglykol mindestens 3, insbesondere 3,3, beträgt. Dieses Verhältnis hat sich im Rahmen der Erfindung als geeignet erwiesen, um hohe Durchsatzraten und hohe Qualität der erhaltenen Recyclingprodukte zu erreichen.

In weiterer vorteilhafter Ausgestaltung des erfindungsgemäßen Verfahrens wird das Alkali- und/oder Erdalkalihydroxid derart mit einem Massenstrom beigefügt, dass das stöchiometrische Verhältnis von Alkali- und/oder Erdalkalihydroxid zu Polyalkylenterephthalat bezogen auf eine konstitutionelle Repetiereinheit mindestens 2, insbesondere etwa 2,4, ist. Insbesondere kann zur Verarbeitung eines Massenstroms von 6,66 kg/h PET-haltiger Abfälle ein Massenstrom von 3,33 kg/h Natriumhydroxid verwendet werden.

In weiterer vorteilhafter Ausgestaltung des erfindungsgemäßen Verfahrens wird das Reaktionsgemisch für die Depolymerisation kontinuierlich durch einen Reaktorbehälter gefördert. Durch einen kontinuierlichen Betrieb kann mit Vorteil ein hoher Durchsatz erzielt werden. Zudem ermöglicht ein kontinuierlicher Durchsatz durch einen Reaktorbehälter eine energieeffiziente Verfahrensweise, da der Reaktorbehälter auf konstante Temperaturwerte eingeregelt werden kann.

Im Rahmen der Erfindung ist es günstig wenn zum Fördern ein Doppelschneckenextruder verwendet wird, wobei vorzugsweise die Schnecken gleichsinnig gedreht werden. Die Verwendung eines gleichsinnig drehenden Doppelschneckenextruders mit dichtkämmenden Schneckenelementen stellt mit Vorteil eine gute Durchmischung des Reaktionsgemisches sicher, insbesondere wenn als Alkali- beziehungsweise Erdalkalihydroxid Natriumhydroxid, etwa in Perlform, verwendet wird. Dabei ist eine hohe mechanische Beanspruchung der Feststoffe angestrebt.

Weiter ist es in vorteilhafter Ausgestaltung des erfindungsgemäßen Verfahrens günstig, wenn die Depolymerisation bei einer Temperatur unterhalb des Zersetzungspunktes des Polyalkylenterephthalats und/oder unterhalb des Siedepunktes von MEG, insbesondere bei 160°C, durchgeführt wird. Gegenüber herkömmlichen Verfahren, welche bei Temperaturen zwischen 180°C -250°C und oberhalb des Siedepunktes des entstehenden Alkylenglykols, also im Falle von PET-Abfällen oberhalb von Temperaturen von 197°C, arbeiten, ist eine energiesparende Verfahrensweise möglich. Da entsprechend nur geringe Drücke erforderlich sind, müssen für die Durchführung des erfindungsgemäßen Verfahrens keine Reaktorbehälter eingesetzt werden, welche, für Hochdruck geeignet sind. Insbesondere können nach der Erfindung Extruder als Reaktorbehälter eingesetzt werden. Der Hauptvorteil eines Extruders ist erfindungsgemäß die kontinuierliche Verfahrensweise und die gute Durchmischung des Produkts.

Im Rahmen des erfindungsgemäßen Verfahrens ist es vorteilhaft, wenn in dem Reaktorbehälter Inertgas, vorzugsweise Stickstoff, eingeleitet wird. Anstelle von Stickstoff kann im Rahmen der Erfindung auch ein Edelgas oder ein Gemisch aus Edelgasen und/oder Stickstoff eingeleitet werden. Diese Maßnahme verhindert das Einströmen von Sauerstoff oder Luftfeuchtigkeit in den Reaktorbehälter, um eine konstante Dosierung zu gewährleisten. Außerdem wird mit einer Intertgasüberlagerung erfindungsgemäß mit Vorteil verhindert, dass das stark hygroskopische Natriumhydroxid verklebt und durch Verblockung den Reaktionsprozess zum Erliegen bringt.

Um eine hohe Recyclingrate bei hohem Durchsatz zu gewährleisten, wird in Ausgestaltung der Erfindung das Reaktionsgemisch während der Depolymerisation geknetet und/oder gemischt und/oder gefördert und/oder rückgefordert. Insbesondere kann in zeitlicher und/oder räumlicher Abfolge eine Abfolge von verschiedenen Knet-, Misch-, Förder- und Rückförderbehandlungen durchgeführt werden, um eine homogene Durchmischung der Feststoffe zu gewährleisten und das PET- Material und die Mehrschichtsysteme mechanisch zu zermahlen und aufzubrechen, um wiederum eine möglichst große Oberfläche für die Verseifungsreaktion bereitzustellen. Durch die mechanische Belastung werden der Materialverbund zwischen den verschiedenen Schichten sowie die Schichten selbst geschädigt, so dass mit Vorteil mit dieser Verfahrensweise eine Reaktion von verschiedenen Seiten an dem PET stattfinden kann. Außerdem kann durch geeignete Auswahl der Abfolge der Behandlungen des Reaktionsgemisches eine gewünschte mittlere Verweilzeit der Abfälle im Reaktionsbehälter eingestellt werden, beispielsweise 2 Minuten.

In bevorzugter Ausgestaltung des erfindungsgemäßen Verfahrens wird aus einem Reaktionsaustrag Alkylenglykol, vorzugsweise durch Verdampfen, entfernt. Zwar können erfindungsgemäß sowohl das als Edukt eingesetzte Alkylenglykol, beispielsweise MEG, als auch das sich bildende Alkylenglykol durch Kondensation zurückgewonnen werden. Dies ermöglicht eine besonders effiziente Verfahrensführung.

Zur Weiterverarbeitung des nach der Depolymerisation erhaltenen Reaktionsaustrags kann in bevorzugter Ausgestaltung des erfindungsgemäßen Verfahrens dem Reaktionsaustrag Wasser zum Lösen fester Bestandteile beigefügt werden. Dies kann in einem Rührkessel oder in einer Mischschnecke erfolgen. Dabei wird die Auflösung des bei der Depolymerisation erhaltenen TPA-Salzes erreicht. Bei der Verarbeitung von PET-haltigen Abfällen unter Beifügung von Natriumhydroxid wird durch die Beifügung von Wasser das bei der Depolymerisation entstandene Dinatriumterephthalat gelöst.

In einer weiteren bevorzugten Ausgestaltungsform des erfindungsgemäßen Verfahrens werden Feststoffe aus dem Reaktionsaustrag herausgefiltert. Dies sind insbesondere unlösliche Reststoffe, wie zum Beispiel PET-Reste, Polyethylen, Polypropylen, Metalle, Pappe oder Polystyrol.

Anschließend kann in weiterer vorteilhafter Ausgestaltung des erfindungsgemäßen Verfahrens dem Reaktionsaustrag eine Säure beigefügt werden, um im Reaktionsaustrag enthaltene bei der Depolymerisation gebildete Carboxylat-Ionen in Säure umzuwandeln. Dazu muss die Säure erfindungsgemäß stärker als die gebildete TPA sein. In diesem Zusammenhang eignet sich erfindungsgemäß insbesondere Schwefelsäure mit einer Konzentration von 25 % (w/w).

Die der Erfindung zu Grunde liegende Aufgabe wird gleichermaßen gelöst durch eine Vorrichtung der eingangs genannten Art zur Durchführung eines Verfahrens zur Wiederaufarbeitung nach einem der Ansprüche 1 bis 13, mit einem Reaktorbehälter, welcher Fördermittel aufweist, sowie mit Mitteln zum Zuführen eines, vorzugsweise festen, Alkali- und/oder Erdalkalihydroxids und mit Mitteln zum Zuführen eines Alkylenglykols in den Reaktorbehälter. Dadurch, dass die erfindungsgemäße Vorrichtung einen Reaktorbehälter mit Fördermitteln umfasst, ist eine kontinuierliche Verfahrensführung möglich.

Wenn der Reaktorbehälter in Ausgestaltung der Erfindung temperiert ist, wird mit den Fördermitteln eine gewünschte Verweilzeit im Reaktorbehälter ermöglicht, welche hohe Durchsatzraten bei hoher Wiederverwertung sicherstellt.

Die Maßnahme gemäß der Erfindung, wonach Mittel zum Zuführen eines Alkylenglykols, beispielsweise MEG, vorgesehen sind, ermöglicht die Durchführung des erfindungsgemäßen Verfahrens, welches sich als besonders geeignet zur Wiederaufarbeitung von Mehrschichtabfällen erwiesen hat.

Die Mittel zum Zuführen eines Alkalihydroxids können eine gravimetrische Dosiereinrichtung mit einem Zwangsförderer umfassen, um beispielsweise festes Natriumhydroxid in Perlform hinzugegeben. Die Mittel zum Zuführen des Hydroxids können als Feststoffdosierer ausgestaltet sein. Ferner können die Mittel zum Zuführen eines Alkylenglykols, beispielsweise MEG, eine gravimetrische Dosiereinheit umfassen.

In Ausgestaltung der erfindungsgemäßen Vorrichtung ist der Reaktorbehälter als Doppelschneckenextruder, vorzugsweise gleichsinnig drehend, ausgeformt. Dadurch kann bei der Durchführung des Verfahrens eine homogene Durchmischung der Feststoffe gewährleistet werden und das wieder aufzuarbeitende Material insbesondere mit Mehrschichtsystemen kann mechanisch zermahlen und aufgebrochen werden, um eine möglichst große Oberfläche für die Verseifungsreaktion bereitzustellen.

In bevorzugter Ausgestaltung der erfindungsgemäßen Vorrichtung weisendie Fördermittel mindestens eine Schneckenanordnung mit mindestens einem Schneckenelement auf, dessen äußerer Durchmesser zum inneren Durchmesser in einem Verhältnis von etwa 1,7, insbesondere 1,66, steht. Dieses Verhältnis hat sich hinsichtlich der Qualität der Wiederaufarbeitung auf der einen Seite und des Durchsatzes auf der anderen Seite im Rahmen der Erfindung als geeignet erwiesen.

Weiter hat es sich als vorteilhaft erwiesen, wenn in Ausgestaltung der erfindungsgemäßen Vorrichtung bei der Schneckenanordnung eine Länge zum äußeren Durchmesser in einem Verhältnis von etwa 60 steht. Hierdurch können Verweilzeiten von lediglich 2 Minuten eingestellt werden, in welchen dennoch eine sehr hohe Umwandlungsrate erzielt wird.

Insbesondere können in Weiterbildung der erfindungsgemäßen Vorrichtung die Fördermittel hintereinander angeordnet fördernde, förderneutrale und/oder rückfördernde Schneckenelemente aufweisen, um das Reaktionsgemisch in dem Reaktor abschnittsweise zu fördern, zu kneten oder rückzufördern. Bei einer geeigneten Abfolge von verschiedenen Knet-, Misch-, Förder- und Rückförderelementen wird erfindungsgemäß eine homogene Durchmischung der Feststoffe gewährleistet und das aufzuarbeitende Polyalkylenterephthalat-Material und die Mehrschichtsysteme werden mechanisch zermahlen und aufgebrochen. Dies ermöglicht eine möglichst große Oberfläche für die Verseifungsreaktion. Dabei schädigt die mechanische Belastung den Materialverbund zwischen den verschiedenen Schichten und die Schichten selber, so dass eine Reaktion von allen Seiten an dem Polyethylenterephthalat stattfinden kann. Durch geeignete Kombination von Schneckenelementen kann erfindungsgemäß die mittlere Verweilzeit des Abfalls im Extruder auf lediglich etwa 2 Minuten eingestellt werden, wobei in dieser kurzen Reaktionszeit ein Umsatz bei der Depolymerisation im Bereich 92-97 % erzielt werden. Die Schneckenelemente können im Rahmen der Erfindung eine Länge von etwa dem einfachen bis doppelten des Durchmessers aufweisen.

Erfindungsgemäß können die verwendeten Schneckenelemente in einer gewünschten Reihenfolge auf eine Welle aufgefädelt werden. Dabei können bei einem Gangzahlwechsel der Schneckenelemente Distanzscheiben oder Übergangselemente eingesetzt werden. Dabei können zur Erreichung einer möglichst hohen mechanischen Beanspruchung und Sicherstellung einer mittleren Verweilzeit von etwa 2 Minuten fördernde und förderneutrale Knetelemente eingesetzt werden. Durch den Einsatz von Knetelementen wird erfindungsgemäß mit Vorteil Energie in die Reaktionsmischung eingetragen, welches die Reaktion beschleunigen kann. Außerdem sorgen erfindungsgemäß die Knetelemente für eine gute Dispergierung der Base im Reaktionsgemisch. Der Einsatz von einem rückfördernden Element führt zu einem Abstauen der Reaktionsmischung. Ein enger Spalt zwischen den rückfördernden Elementen erzwingt erfindungsgemäß das Verweilen der Reaktionsmischung, bis die Abfallreste durch den Spalt zwischen Elementen und der Zylinderwand gepresst werden können. Wenn erfindungsgemäß einige Schneckenelemente als fördernde Mischelemente ausgestaltet sind, wird eine sehr gute Durchmischung erreicht bei geringer Scherung, welche das Reaktionsprodukt weniger stark mechanisch belastet als Knetelemente.

In vorteilhafter Weiterbildung der erfindungsgemäßen Vorrichtung ist der Reaktorbehälter mit Mitteln zur an die Schneckenelemente angepassten abschnittsweisen Temperierung versehen. Diese Maßnahme ermöglicht es im Rahmen der Erfindung mit Vorteil, eine für die jeweilige mechanische Behandlung angepasste Temperaturführung zu wählen. Dazu können einzelne Gehäuseabschnitte des Reaktionsbehälters erfindungsgemäß jeweils mit einer individuell ansteuerbaren elektrischen Heizung und einer Wasserkühlung ausgestaltet sein.

Schließlich wird die der Erfindung zu Grunde liegende Erfindung durch eine Verwendung einer Vorrichtung nach einem der Ansprüche 14 bis 19 zur Durchführung eines Verfahrens nach einem der Ansprüche 1 bis 13 gelöst.

Es werden bevorzugt mit dem erfindungsgemäßen Verfahren Abfälle aus Polyalkylenterephthalat als Duo- und/oder Mehrschichtsysteme mit einem Polymer oder mehreren verschiedenen Polymeren und/oder Naturfasern und/oder Metallbeschichtungen verwendet. Bevorzugt weisen die Polyalkylenterephthalat enthaltenden Abfälle eine Schicht aus Polyethylenterephthalat auf. Dies sind beispielsweise die handelsüblichen PET-Flaschen oder Verpackungen für Lebensmittel.

Im Gegensatz zur lösungsmittelfreien Verarbeitung von reinen oder mit anderen Polymeren gemischten Polyalkylenterephthalat-Abfällen gemäß DE 69316545 T2 ist es im erfindungsgemäßen Verfahren möglich, beschichtete Polyalkylenterephthalat-Abfälle und Polyalkylenterephthalat enthaltende Mehrschichtsysteme zu verarbeiten. Für bestimmte Anwendungen ist es im Rahmen der Erfindung vorteilhaft, wenn in dem Extruder oder in dem Knetreaktor ein Lösungsmittel oder ein Lösungsmittelgemisch zugegeben wird. Dabei ist das Lösungsmittel bevorzugt aus der Gruppe der Alkohole ausgewählt.

Durch die Lösungsmittelbehandlung des Materials und die Zugabe von Lösungsmittel im Depolymerisationsprozess im Extruder oder Knetreaktor werden eine bessere Durchmischung, ein besserer Phasenkontakt sowie ein erhöhter Stoffübergang gewährleistet und die Depolymerisationseffektivität erhöht.

Der Polyalkylenterephthalat enthaltende Abfall, der beispielsweise aus Flaschen-, Film-, Faser-, Schalen-, Automobilinnenraumverkleidung, und anderen Verpackungsabfällen besteht, wird gemäß der Erfindung vor der Behandlung zerkleinert und kontinuierlich in einem Reaktor mit einem Alkalimetall- oder Erdalkalimetallhydroxid gemischt. Die Reagenzien werden in einer Weise zugeführt, dass das Alkalimetall- oder Erdalkalimetallhydroxid in einem stöchiometrischen oder in einem leichten stöchiometrischen Überschuss gegenüber der konstitutionellen Repetiereinheit des Polyalkylenterephthalats vorliegt. Bei dem verwendeten Reaktor kann es sich nach der Erfindung um einen kontinuierlich arbeitenden Extruder oder Knetreaktor handeln.

Bei dem Verfahren nach der Erfindung kann es vorteilhaft sein, alle zugeführten Reagenzien und den zerkleinerten, Polyalkylenterephthalat enthaltenden Abfall vor und während der Verarbeitung im Extruder oder Knetreaktor mit einer Inertgasatmosphäre zu überlagern oder zu überströmen. Diese Inertgasatmosphäre kann aus Stickstoff, Edelgasen oder Gemischen derselben und in besonderen Verfahrensweisen aus trockener oder synthetischer Luft bestehen.

Für eine gute Durchmischung der Materialien kann in Ausgestaltung der Erfindung eine gleichsinnig oder gegenläufig rotierende, dicht kämmende Doppelwellenschnecke oder ein Mehrwellenextruder, sowie ein Knetreaktor mit, vorzugsweise selbstreinigenden Schaufeln, verwendet werden. Die Anordnung der Extrusionsschneckenelemente sowie die Anordnung der Schaufeln ist vorteilhaft selbstreinigend ausgeführt und kann durch den Einsatz von verschiedenen Misch-, Förder-, Rückförder- und Knetelementen an den Prozess angepasst werden.

Die Extrusionsschneckenelemente können in dem Verfahren in Ausgestaltung der Erfindung so angeordnet sein, dass das entstehende Alkylenglykol bei vermindertem Druck oder durch das Überströmen mit Inertgas entfernt werden kann. Die Lösungsmittel- und Alkylenglykoldämpfe können in bevorzugter Ausgestaltung der Erfindung außerhalb des Reaktors durch geeignete Verfahren, wie z.B. Kondensieren, gewonnen werden.

In einer anderen erfindungsgemäßen Verfahrensvariante können die Schaufeln des Knetmischers so angeordnet sein, dass sie selbstreinigend das Gemisch in einer Weise homogenisieren und die Verseifung der Polyalkylenterephthalatanteile im Abfall innerhalb von 1-60 min durchgeführt werden kann. Der Reaktor kann auch in dieser erfindungsgemäßen Variante mit einem Inertgas durchströmt werden, welches die Alkylenglykoldämpfe aus dem Reaktor herausschleppt. Diese Dämpfe können im Rahmen der Erfindung außerhalb des Reaktors durch geeignetes Equipment zurückgewonnen werden.

Als Reaktionsaustrag werden nach dem erfindungsgemäßen Verfahren ein Alkalimetall- oder Erdalkalimetallterephthalat, ein Alkylenglykol sowie das optional eingesetzte Lösungsmittel erhalten. Das Alkalimetall- oder Erdalkalimetallterephthalat wird im nächsten Verfahrensschritt in einem geeigneten Lösungsmittel, vorzugsweise Wasser gelöst, filtriert und gereinigt. Bei der Filtration können so auf einfache Weise die Beschichtungen, die während des Verfahrens zum Teil unverändert hervorgehen, aus den Mehrschichtsystemen wiedergewonnen werden. In einem konkreten Beispiel gemäß der Erfindung können dies PE- oder andere Polyolefin-Anteile sein, die in einem PE/PET- oder PP/PET-Mehrschichtsystem als Lebensmittelverpackung in den Abfall gelangt sind.

Im Gegensatz zu den Verfahren gemäß Stand der Technik, die zur Bildung von Alkalimetall- oder Erdalkalimetallterephthalaten aus nicht beschichteten PET-Abfällen führen, erlaubt das erfindungsgemäße Verfahren die Verarbeitung von beschichteten und mehrlagigen Polyalkylenterephthalat enthaltenden Abfällen und Gemischen von verschiedenen Polymeren und Polyalkylenterephthalat und Abfällen und die Herstellung von wertvollen Alkalimetall- oder Erdalkalimetallterephthalaten. Aus den erhaltenen Alkalimetall- oder Erdalkalimetallterephthalaten kann in wässriger Lösung durch Zugabe einer stärkeren Säure als TPA die TPA zurückgewonnen werden.

Die Entwicklung in der letzten Dekade hat gezeigt, dass dringend eine Recyclingmöglichkeit für die großen Mengen an Verpackungsmaterial gefunden werden muss. Das Verfahren nach der Erfindung kann für einen wesentlichen Teil dieses Problems eine Lösung bieten, da mit dem erfindungsgemäßen Verfahren insbesondere die ein- und mehrschichtigen Polyethylenterephthalat enthaltenden Flaschen oder andere Flüssigkeitsbehälter, Verpackungsschalen und Folien verwertet werden können, was nach dem Stand der Technik nicht möglich ist, sobald ein direkter Materialverbund zwischen zwei oder mehr verschiedenen Materialien besteht.

Neben dem direkten Materialverbund toleriert das erfindungsgemäße Verfahren mit Vorteil im Polyalkylenterephthalat enthaltenden Abfall auch Verunreinigungen wie beispielsweise Additive, Füllstoffe, Färbemittel, Pigmente, Umhüllungen, Etiketten, Metalle und Metallbeschichtungen und dergleichen. Die Verunreinigungen können im Rahmen der Erfindung durch Filtration und/oder andere Verfahrensstufen abgetrennt werden, nachdem der Reaktionsaustrag, die Alkalimetall- oder Erdalkalimetallterephthalate, in Wasser gelöst worden sind. Das Zielprodukt, die TPA, wird nach einem Reinigungsschritt durch das Senken des pH-Wertes mit einer stärkeren Säure als TPA erhalten. Nachfolgend werden zur näheren Erläuterung des Verwertungsverfahrens Anwendungsbeispiele beschrieben, ohne sie jedoch darauf zu beschränken.

### Beispiel 1

In einen gleichläufigen Doppelschneckenextruder mit einem Schneckendurchmesser von 18 mm gibt man unter Inertgasatmosphäre kontinuierlich mit Hilfe von zwei Dosiereinrichtungen 0,8 kg/h PE beschichtete PET-Flakes und 0,4 kg/h Natriumhydroxid. Diese Zugabeströme erlauben die Aufrechterhaltung eines konstanten Gewichtsverhältnisses PET/NaOH von etwa 2, bezogen auf die konstitutionelle Repetiereinheit von PET. Die Gehäusetemperatur des Extruders wird zwischen 160-180°C eingestellt. Die Drehzahl der Doppelschnecke beträgt 500 U/min. Die Entnahme einer Probe des Produkts zeigt einen PET-Verseifungsgrad von >80%. Im Doppelschneckenextruder wird das entstehende MEG destillativ entfernt. Der so erhaltene Feststoff besteht im Wesentlichen aus Mono- und Dinatriumterephthalat sowie nicht reagierten PE-Anteilen. Der Extruderaustrag wird in Wasser gelöst und anschließend einer Fest-Flüssig-Trennung unterzogen, bevor die Lösung gereinigt und die TPA mit Hilfe einer starken Säure ausgefällt wird.

### Beispiel 2

Im gleichen Gerät wie in Beispiel 1 unter Anwendung eines ähnlichen Verfahrens behandelt man einen heterogenen Inputstrom aus Abfällen, in dem unter anderem mit PE beschichtetes PET sowie weitere Polymere, insbesondere Polyolefine wie PP, enthalten sind. In dem Inputstrom sind etwa 0,8 kg/h PP/PE beschichtete PET-Flakes enthalten, die 0,4 kg/h Natriumhydroxid unter Zugabe von 0,9 kg/h MEG werden jeweils getrennt dosiert in den Extruder eingebracht. Diese Zugabeströme erlauben die Aufrechterhaltung eines konstanten Gewichtsverhältnisses PET/NaOH von etwa 2. Die gesamte Apparatur wird dabei mit Inertgas überlagert. Die Gehäusetemperatur des Extruders wird zwischen 140-160°C eingestellt. Die Drehzahl der Doppelschnecke beträgt 400 U/min. Die Entnahme einer Probe des Produkts zeigt einen PET-Verseifungsgrad von >90%. Im Doppelschneckenextruder werden das eingesetzte sowie das entstehende MEG bei vermindertem Druck entfernt. Der so erhaltene Feststoff besteht im Wesentlichen aus Mono- und Dinatriumterephthalat sowie nicht reagierten Polyolefinanteilen insbesondere aus PP- und PE-Anteilen.

### Beispiel 3

In einem ähnlichen Gerät wie in Beispiel 1 mit einem Schneckendurchmesser von 27 mm unter Anwendung eines ähnlichen Verfahrens behandelt man 5 kg/h PE beschichtete PET-Flakes mit 2,5 kg/h Natriumhydroxid unter Zugabe von 5,7 kg/h MEG. Diese Zugabeströme erlauben die Aufrechterhaltung eines konstanten Gewichtsverhältnisses PET/NaOH von etwa 2. Die Gehäusetemperatur des Extruders wird zwischen 140-160°C eingestellt. Die Drehzahl der Doppelschnecke beträgt 270 U/min. Die Entnahme einer Probe des Produkts zeigt einen PET-Verseifungsgrad von >90%. Im Doppelschneckenextruder werden das eingesetzte sowie das entstehende MEG destillativ entfernt. Der so erhaltene Feststoff besteht im Wesentlichen aus Mono- und Dinatriumterephthalat sowie nicht reagierten PE-Anteilen.

### Beispiel 4

In einem Doppelwellenknetreaktor gibt man kontinuierlich mit Hilfe von drei Dosiereinrichtungen 0,8 kg/h PE beschichtete PET-Flakes und 0,4 kg/h Natriumhydroxid unter Zugabe von 0,9 kg/h MEG. Wobei diese Zugabemengen getrennt in den Doppelwellenknetreaktor dosiert werden und die konstante Aufrechterhaltung eines stöchiometrischen Verhältnisses NaOH/PET von etwa 2,4 erlauben, bezogen auf die konstitutionelle Repetiereinheit von PET. Die Gehäusetemperatur des Knetreaktors wird zwischen 160-180°C eingestellt. Die Drehzahl der Knetwelle beträgt 500 U/min. Die Entnahme einer Probe des Produkts zeigt einen PET-Verseifungsgrad von >80%. Im Doppelwellenknetreaktor werden das eingesetzte sowie das entstehende MEG destillativ entfernt. Der so erhaltene Feststoff besteht im Wesentlichen aus Mono- und Dinatriumterephthalat sowie nicht reagierten PE-Anteilen.

Nachfolgend sind weitere Merkmale der Erfindung aufgeführt.

Merkmal 1. Verfahren zum Recycling von Polyalkylenterephthalat enthaltenden Abfällen mit den Schritten:
- Zerkleinern der Abfälle,
- Zuführen der zerkleinerten Abfälle sowie eines Alkalimetall- oder Erdalkalimetallhydroxids in einen Extruder oder in einen Knetreaktor,
- Mischen und Erwärmen der zerkleinerten Abfälle mit dem Alkalimetall- oder Erdalkalimetallhydroxid im Extruder oder Knetreaktor zur Bildung einer Verseifung und
- Austrag eines dabei entstehenden Alkalimetall- oder Erdalkalimetallterephthalat enthaltenden Zwischenproduktes.

Merlmal 2. Verfahren nach Merkmal 1, dadurch gekennzeichnet, dass die Polyalkylenterephthalat enthaltenden Abfälle Duo- und/oder Mehrschichtsysteme mit einem Polymer oder mehreren verschiedenen Polymeren sind.

Merkmal 3. Verfahren nach Merkmal 1 oder 2, dadurch gekennzeichnet, dass die Polyalkylenterephthalat enthaltenden Abfälle andere Polymere und/oder Gemische anderer Polymere und/oder Naturstoffe und/oder Metalle enthalten.

Merkmal 4. Verfahren nach Merkmal 1, 2 oder 3, dadurch gekennzeichnet, dass die Polyalkylenterephthalat enthaltenden Abfälle eine oder mehrere Schichten aus Ethylen-Vinylalkohol-Copolymer (EVOH), Pappe, Ethylen-Vinylacetat-Copolymer (EVA), Polyvinylalkohol (PVOH) Polyamid (PA), Polyethylen (PE), Polypropylen (PP), Polystyrol (PS) oder deren Copolymere sowie Metalle sowie deren Gemische enthalten.

Merkmal 5. Verfahren nach einem der vorangehenden Merkmale 1 bis 4, dadurch gekennzeichnet, dass die Polyalkylenterephthalat enthaltenden Abfälle eine Schicht aus Polyethylenterephthalat aufweisen.

Merkmal 6. Verfahren nach einem der vorangehenden Merkmale, dadurch gekennzeichnet, dass in den Extruder oder in den Knetreaktor ein Lösungsmittel oder ein Lösungsmittelgemisch zugegeben wird.

Merkmal 7. Verfahren nach Merkmal 6, dadurch gekennzeichnet, dass das Lösungsmittel aus der Gruppe der Alkohole ist, oder dass das Lösungsmittel ein unpolares, halogeniertes Lösungsmittel ist, insbesondere Dichlormethan, Chloroform, Tetrachlormethan, 1,2 Dichlorethan, oder dass das Lösungsmittel ein nicht halogeniertes Lösungsmittel ist, insbesondere Dimethylsulfoxid, oder dass das Lösungsmittel 1,4-Dioxan oder Tetrahydrofuran ist.

Merkmal 8. Verfahren nach einem der vorangehenden Merkmale, dadurch gekennzeichnet, dass zur Verseifung Zinkacetat, Natriumcarbonat, Natriumhydrogencarbonat, Zinkchlorid und/oder Bleiacetat als Katalysatoren zugegeben werden.

Merkmal 9. Verfahren nach einem der vorangehenden Merkmale, dadurch gekennzeichnet, dass die Reaktivextrusion oder Knetreaktion bei

Temperaturen von 100° C bis 180° C, bevorzugt von 140°C bis 160°C, durchgeführt wird.

Merkmal 10. Verfahren nach einem der vorangehenden Merkmale, dadurch gekennzeichnet, dass die Reaktivextrusion oder Knetreaktion kontinuierlich und unter Überlagerung mit Inertgas, insbesondere Argon/ Stickstoff, für eine trockene und sauerstofffreie Atmosphäre durchgeführt wird.

Merkmal 11. Verfahren nach einem der vorangehenden Merkmale, dadurch gekennzeichnet, dass das bei der Verseifung entstehende Alkylenglykol destillativ abgetrennt wird.

Die Erfindung wird in einer bevorzugten Ausführungsform unter Bezugnahme auf eine Zeichnung beispielhaft beschrieben, wobei weitere vorteilhafte Einzelheiten den Figuren der Zeichnung zu entnehmen sind.

Funktionsmäßig gleiche Teile sind dabei mit denselben Bezugszeichen versehen.

Die einzige Figur der Zeichnung zeigt im Einzelnen:
- Figur 1:: Blockfließbild zur Veranschaulichung der Verfahrensschritte einer Ausgestaltungsform des erfindungsgemäßen Verfahrens.

Die mit Bezugnahme auf die Fig. 1 beschriebene bevorzugte Ausführungsform des erfindungsgemäßen Verfahrens ermöglicht das Recycling von bisher nicht - oder nur thermisch - verwertbaren Polyethylenterephthalat (PET)-Abfällen. Das Verfahren kann auch für die Verwertung von anderen Polyalkylenterephthalaten wie z.B Polybutylenterephthalat verwendet werden.

PET-haltige Abfälle, auch Mehrschichtsysteme, wie z.B. Getränkeflaschen, Waschmittelflaschen (opak, klar oder schwarz gefärbt) oder Lebensmittelverpackungen anderer Art z.B. Salatschalen, Wurst- und Käseverpackungen oder PET-haltige Produktionsabfälle werden in einem ersten Schritt 1 gewaschen und auf kleiner 3mm zerkleinert. Anschließend werden die Abfälle optional in einem zweiten Schritt 2 vorgetrocknet, um den Wassergehalt des PET-Materials zu reduzieren. Alternativ kann das zu bearbeitende Material nach dem erfindungsgemäßen Verfahren vorgetrocknet werden. In diesem Falle kann der Schritt 2 der Trocknung im Nachgang zu dem Schritt 1 des Zerkleiners entfallen. Bei bestimmten Anwendungsfällen nach der Erfindung kann jedoch eine weitere intensivere Trocknung 2 vorteilhaft sein.

In einem weiteren Prozessschritt "Depolymerisation" 3 werden die Abfälle in einen gleichsinnig drehenden Doppelschneckenextruder mit dicht kämmenden Schneckenelementen eingeführt. In dem Extruder wird die Verseifungs- bzw. Depolymerisationsreaktion des PET kontinuierlich durchgeführt. Es werden in der mit Bezug auf die Fig. 1 beispielhaft beschriebenen Anlage 6,66 kg/h PET-haltige Abfälle, 3,33 kg/h Natriumhydroxid und 2 kg/h MEG im Extruder verarbeitet. Das Verhältnis aus Natriumhydroxid zu PET-Abfällen wird erfindungsgemäß während des Prozesses so eingestellt, dass sich ein konstantes stöchiometrisches Verhältnis von etwa 2,4, bezogen auf die konstitutionelle Repetiereinheit von PET, einstellt. Der Reaktionsaustrag des Extruders besteht aus Dinatriumterephthalat, MEG und nicht reagierten Anteilen des Natriumhydroxids und des PET-Abfalls, wie z.B. PET-Reste, Farbstoffe, Abbauprodukten von PA und Farbstoffen, anderen Polymeren wie z.B. PE, PP und PS.

Der Doppelschneckenextruder ist modular aufgebaut und besteht aus 14 Temperaturzonen. Die Gehäuse sind je mit einer individuell ansteuerbaren elektrischen Heizung und einer Wasserkühlung ausgestattet. Das Verhältnis des äußeren Schneckendurchmessers Da zum inneren Schneckendurchmesser Di ist eine Kenngröße für das mögliche, freie Schneckenvolumen. Bei dem eingesetzten Extruder beträgt das Da/Di der Schneckenelemente 1,66. Das Verhältnis der Schneckenlänge L zum Durchmesser der Schnecke D beschreibt die Verfahrenslänge des Extruders und beträgt bei diesem Extruder 60. Die Schneckengeometrie ist modular aufgebaut und kann an den Prozess und das PET-Material angepasst werden. Der Extruder besteht in aus folgenden individuell temperierbaren Zylindern:
Zylinder 1: Haupteinzug, Zylinder 2: Einspritzdüse oben, Zylinder 3:
   Seitenentgasung mit Rückförderung, Zylinder 4: Seitenbeschickung des
Natriumhydroxids, Zylinder 5: Einsprühdüse oben, Zylinder 6:
   Entlüftungsstutzen oben, Zylinder 7: geschlossen, Zylinder 8:
   Einsprühdüse oben, Zylinder 9: Seitenentgasung mit Rückförderung,
Zylinder 10: geschlossen, Zylinder 11: Entgasung, Zylinder 12:
   geschlossen, Zylinder 13: Entgasung, Zylinder 14: Einsprühdüse,
Zylinder 15: Förderung, Austrag hinter Zylinder 15. In den Zylindern 1 bis 15 werden die Edukte somit zunächst in den Extruder eingezogen und im Gerät beim Durchlaufen aller Zonen mechanisch bearbeitet. Im letzten, also fünfzehnten, Zylinder wird das Produkt aus dem Extruder herausgefördert. Der Austrag ist als Öffnung, aus der das Produkt aus dem Gerät herausgefördert wird, ausgestaltet.

Das Gerät ist mit bis zu drei Drucksensoren ausgestattet, die in die Zylinderöffnung der Einsprühdüse eingesetzt sind. Die Gehäuse/Zylinder 2 bis 15 werden auf 160°C temperiert. Zylinder 1 wird nicht temperiert. Die Drehzahl der gleichsinnig drehenden Doppelschnecke wird auf 100 U/min eingestellt.

Die Schneckenkonfiguration ist so ausgewählt, sodass im Prozess eine gute Durchmischung der beiden Feststoffe gewährleistet werden kann. Die verwendeten Schneckenelemente können in einer beliebigen Reihenfolge auf die Welle aufgefädelt werden. Bei einem Gangzahlwechsel der Schneckenelemente werden Distanzscheiben oder Übergangselemente eingesetzt. Um eine möglichst hohe Deformation/ mechanische Beanspruchung und eine relativ hohe mittlere Verweilzeit von etwa 2 Minuten der mehrschichtigen PET-Abfälle zu erreichen, werden bei der Konstruktion der Schneckenkonfiguration fördernde und förderneutrale Knetelemente eingesetzt. Des Weiteren wird durch den Einsatz von Knetelementen Energie in die Reaktionsmischung eingetragen, die so die Reaktion beschleunigen kann. Des Weiteren sorgen Knetelemente für eine gute Dispergierung der Base im Reaktionsgemisch. Der Einsatz von einem rückfördernden Element führt zu einem Abstauen der Reaktionsmischung. Der enge Spalt zwischen den rückfördernden Elementen erzwingt das Verweilen der Reaktionsmischung, bis die PET-Abfall-Reste durch den Spalt zwischen Elementen und der Zylinderwand gepresst werden können. Im Bereich der Entgasung und atmosphärischen Öffnung werden Schneckenelemente mit einem hohen freien Schneckenvolumen eingesetzt. Dies erlaubt das kontinuierliche Entfernen von Lösungsmittel aus der Reaktionsmischung. Weiterhin sind in der Schneckenkonfiguration eine Reihe von fördernden Mischelementen eingebaut, die durch die geringe Scherung das Reaktionsprodukt weniger stark mechanisch belasten als Knetelemente, jedoch für eine sehr gute Durchmischung sorgen.

Im Bereich des Zylinder 1 wird das PET über einen Feststoffdosierer gravimetrisch dosiert. Über den Einzug und die Schneckenelemente mit einem großen freien Schneckenvolumen wird das Material in den Extruder transportiert und dort erwärmt. In Zylinder 1 selber erfolgt hingegen nur ein Transport, jedoch keine Temperierung. In Zylinder 2 wird über die Einfüllöffnung oben MEG mit Hilfe einer gravimetrischen Dosierung hinzugegeben. Über die Seitendosierung in Zylinder 4 wird festes Natriumhydroxid in Perlform gravimetrisch über eine zweite Dosiereinrichtung mit Hilfe eines Zwangsförderers hinzugegeben. Der Zylinder 4 weist außerdem eine atmosphärische Öffnung auf. Sowohl der Feststoffdosierer für das PET als auch der Feststoffdosierer für das Natriumhydroxid sind mit Inertgas überlagert, um das Einströmen von Sauerstoff und (Luft-)Feuchtigkeit zu verhindern und um eine konstante Dosierung zu gewährleisten. Ohne eine Inertgasüberlagerung würde das stark hygroskopische Natriumhydroxid sehr schnell verkleben und verblocken, was zum Erliegen des Prozesses führen würde. Über eine atmosphärische Öffnung in Zylinder 6 sowie Zylinder 10 kann das eingesetzte MEG und das sich bildende MEG durch Kondensation zurückgewonnen werden.

Die Schneckenkonfiguration ist in Tabelle 1 dargestellt. Die angegebenen Winkelgradwerte bezeichnen jeweils den Winkel zwischen den Scheiben der Knetelemente. Es wird eine Abfolge von verschiedenen Knet-, Misch-, Förder- und Rückförderelementen verwendet, die eine homogene Durchmischung der Feststoffe gewährleisten und das PET-Material und die Mehrschichtsysteme mechanisch zermahlen und aufbrechen, um eine möglichst große Oberfläche für die Verseifungsreaktion bereitzustellen. Durch die mechanische Belastung werden der Materialverbund zwischen den verschiedenen Schichten sowie die Schichten selbst geschädigt, sodass mit dieser Verfahrensweise eine Reaktion von allen/verschiedenen Seiten an dem PET stattfinden kann. Im Gegensatz dazu würde ohne mechanischen Beanspruchung der ein- oder mehrseitig beschichteten PET-Flakes der Angriff durch die Base lediglich an den freiliegenden PET-Oberflächen und PET-Kanten erfolgen. Durch die Auswahl der dargestellten Schneckenkonfiguration wird die mittlere Verweilzeit des PET-Abfalls im Extruder auf etwa 2 min eingestellt. In dieser Reaktionszeit findet ein Umsatz des PET-Anteils im PET-haltigen Abfall zu 92-97% statt.

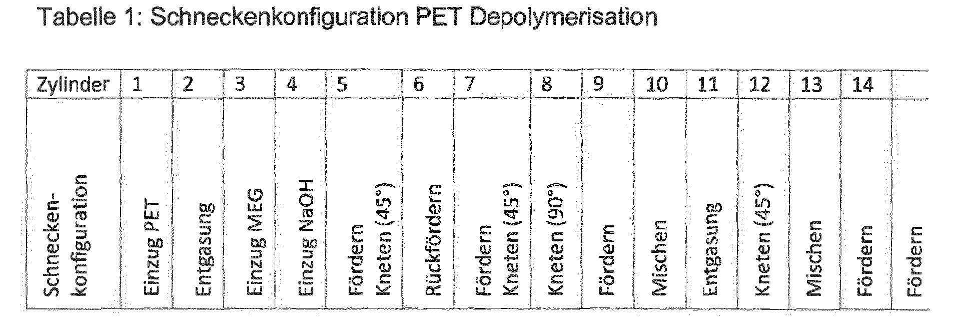

Der pastöse Reaktionsaustrag wird im folgenden Schritt "Nachbehandlung" 4 granuliert, zerkleinert und auf ein temperiertes Förderband mit Absaugung ausgebracht. Die MEG-Dämpfe werden an einem Kühler kondensiert und aufgefangen.

Im folgenden Verfahrensschritt "Lösen" 5 wird der Reaktionsaustrag in einem Rührkessel oder einer Mischschnecke in Wasser (55 kg/h, 133 g/L Löslichkeit des Dinatriumterephthalats) gelöst. Die unlöslichen Reststoffe (PET-Reste, PE, PP, Metalle, PS, Pappe) werden durch Filtration 6 abgetrennt.

Im Anschluss an die Filtration 6 werden in einem Verfahrensschritt "Aufreinigung" 11 Verunreinigungen und Nebenprodukte des Verfahrens abgetrennt. Denkbar sind hier im Rahmen der Erfindung verschiedene Verfahren, welche dem Fachmann für sich genommen bekannt sind.

Im darauffolgenden Verfahrensschritt "TPA-Fällung" 7 wird die Lösung mit Schwefelsäure (9,6 kg/h, 25%(w/w)) versetzt. Die ausgefallene TPA wird durch Filtration 8 gewonnen und mit Wasser gewaschen 9 und abfiltriert. Das TPA wird mit Wasser gewaschen, um Reste der Schwefelsäure und des Natriumsulfats, das sich bei der Fällung bildet, zu entfernen.

Im Anschluss an das Waschen 10 erfolgt eine Trennung Feststoff-Flüssigkeit 10, um das feste TPA, welches in Wasser unlöslich ist, vom Waschwasser zu trennen.

Mit dem erfindungsgemäßen Verfahren, der erfindungsgemäßen Vorrichtung gleichermaßen wie mit der erfindungsgemäßen Verwendung lassen sich insbesondere mehrschichtige PET-haltige Abfälle mit hohem Durchsatz und hoher Qualität in die Ausgangsstoffe der Polymerisation auf effiziente Weise umwandeln, welche uneingeschränkt der Wiederverwertung, also der Herstellung von Polyalkylenterephthalaten, zur Verfügung stehen. Ein Teil des dabei anfallenden Alkylenglykols kann dabei bei dem erfindungsgemäßen Verfahren im Rücklauf für die Depolymerisation verwendet werden.

## Patentansprüche

1. Verfahren zur Wiederaufarbeitung von im wesentlichen Polyalkylenterephthalat, insbesondere Polyethylenterephtalat und/oder Polybutylenterephtalat, enthaltenden Abfällen in einem kontinuierlichen Prozess mittels einer Depolymerisation, wobei zur Herstellung eines Reaktionsgemisches den Abfällen ein, vorzugsweise festes, Alkali- und/oder Erdalkalihydroxid, insbesondere Natriumhydroxid, beigefügt wird, **dadurch gekennzeichnet, dass** dem Reaktionsgemisch als Edukt zusätzlich ein Alkylenglykol beigefügt wird, wobei das Alkylenglykol ein als Produkt der angestrebten Depolymerisation herstellbares Alkylenglykol, insbesondere Monoethylenglykol, ist und wobei dem Reaktionsgemisch keine weiteren reaktiven Bestandteile beigefügt werden, wobei zum Fördern ein Doppelschneckenextruder verwendet wird, wobei vorzugsweise die Schnecken gleichsinnig gedreht werden.

2. Verfahren nach Anspruch 1, **dadurch gekennzeichnet, dass** die Abfälle vor der Herstellung des Reaktionsgemisches, vorzugsweise auf eine Größe von höchstens 3mm, zerkleinert werden.

3. Verfahren nach Anspruch 1 oder 2, **dadurch gekennzeichnet, dass** das Alkylenglykol mit einem Massenstrom beigefügt wird, welcher derart gewählt wird, dass das Massenstromverhältnis von den Abfällen zu Alkylenglykol mindestens drei, insbesondere etwa 3,3, beträgt.

4. Verfahren nach einem der vorhergehenden Ansprüche, **dadurch gekennzeichnet, dass** das Alkali-und/oder Erdalkalihydroxid derart mit einem Massenstrom beigefügt wird, sodass das Verhältnis von Alkali- und/oder Erdalkalihydroxid zu Polyalkylenterephthalat bezogen auf eine konstitutionelle Repetiereinheit etwa stöchiometrisch, insbesondere etwa 2,4, ist.

5. Verfahren nach einem der vorhergehenden Ansprüche, **dadurch gekennzeichnet, dass** das Reaktionsgemisch für die Depolymerisation kontinuierlich durch einen Reaktorbehälter gefördert wird, wobei vorzugsweise das Reaktionsgemisch während der Depolymerisation geknetet und/oder gemischt und/oder gefördert und/oder rückgefördert wird.

6. Verfahren nach einem der vorhergehenden Ansprüche, **dadurch gekennzeichnet, dass** die Depolymerisation bei einer Temperatur unterhalb des Siedepunktes des Polyalkylenterephthalats und/oder unterhalb des Siedepunktes von Monoethylenglykol, insbesondere bei 160°C, durchgeführt wird.

7. Verfahren nach einem der vorhergehenden Ansprüche, **dadurch gekennzeichnet, dass** in den Reaktorbehälter Inertgas, vorzugsweise Stickstoff, eingeleitet wird.

8. Verfahren nach einem der vorhergehenden Ansprüche, **dadurch gekennzeichnet, dass** aus einem Reaktionsaustrag Alkylenglykol, vorzugsweise durch Verdampfen, entfernt wird.

9. Verfahren nach einem der vorhergehenden Ansprüche, **dadurch gekennzeichnet, dass** dem Reaktionsaustrag Wasser zum Lösen fester Bestandteile beigefügt wird, wobei vorzugsweise Feststoffe aus dem Reaktionsaustrag herausgefiltert werden.

10. Verfahren nach einem der vorhergehenden Ansprüche, **dadurch gekennzeichnet, dass** dem Reaktionsaustrag eine Säure beigefügt wird, um im Reaktionsaustrag enthaltene bei der Depolymerisation gebildete Carboxylat-Ionen in Säure umzuwandeln.

11. Vorrichtung zur Durchführung eines Verfahrens zur Wiederaufarbeitung nach einem der Ansprüche 1 bis 10, mit einem Reaktorbehälter, welcher Fördermittel aufweist, sowie Mitteln zum Zuführen eines, vorzugsweise festen, Alkali- und/oder Erdalkalihydroxids und Mitteln zum Zuführen eines Alkylenglykols in den Reaktorbehälter, wobei
der Reaktorbehälter als Doppelschneckenextruder, vorzugsweise gleichsinnig drehend, ausgeformt ist.

12. Vorrichtung nach Anspruch 11, **dadurch gekennzeichnet, dass** der Extruder mindestens ein Schneckenelement aufweist, dessen äußerer Durchmesser zum inneren Durchmesser in einem Verhältnis von etwa 1,7 steht und/oder
dass bei dem Schneckenelement eine Länge zum äußeren Durchmesser in einem Verhältnis von etwa 60 steht.

13. Vorrichtung nach einem der Ansprüche 11 bis 12, **dadurch gekennzeichnet, dass** die Fördermittel hintereinander angeordnet fördernde, förderneutrale und/oder rückfördernde Schneckenelemente aufweisen, um das Reaktionsgemisch in dem Reaktor abschnittweise zu fördern, zu kneten und/oder rückzufördern,.

14. Vorrichtung nach Anspruch 13, **dadurch gekennzeichnet, dass** der Reaktorbehälter mit Mitteln zur an die Schneckenelemente angepassten abschnittweisen Temperierung versehen ist.

15. Verwendung einer Vorrichtung nach einem der Ansprüche 11 bis 14 zur Durchführung eines Verfahrens nach einem der Ansprüche 1 bis 10.

## Claims

1. Method for reprocessing waste substantially containing polyalkylene terephthalate, in particular polyethylene terephthalate and/or polybutylene terephthalate, in a continuous process by means of depolymerisation, a preferably solid alkali hydroxide and/or alkaline earth hydroxide, in particular sodium hydroxide, being added to the waste to produce a reaction mixture, **characterised in that** an alkylene glycol is additionally added to the reaction mixture as a starting material, the alkylene glycol being an alkylene glycol that can be produced as a product of the desired depolymerisation, in particular monoethylene glycol, and no further reactive constituents being added to the reaction mixture, a twin-screw extruder being used for conveying, the screws preferably being rotated in the same direction.

2. Method according to claim 1, **characterised in that** the waste is comminuted, preferably to a size of at most 3 mm, before the reaction mixture is produced.

3. Method according to either claim 1 or claim 2, **characterised in that** the alkylene glycol is added at a mass flow which is selected such that the mass flow ratio of the waste to alkylene glycol is at least three, in particular approximately 3.3.

4. Method according to any of the preceding claims, **characterised in that** the alkali hydroxide and/or alkaline earth hydroxide is added at a mass flow such that the ratio of alkali hydroxide and/or alkaline earth hydroxide to polyalkylene terephthalate, based on a constitutional repeating unit, is approximately stoichiometric, in particular approximately 2.4.

5. Method according to any of the preceding claims, **characterised in that** the reaction mixture for the depolymerisation is continuously conveyed through a reactor vessel, the reaction mixture preferably being kneaded and/or mixed and/or conveyed and/or returned during the depolymerisation.

6. Method according to any of the preceding claims, **characterised in that** the depolymerisation is carried out at a temperature below the boiling point of the polyalkylene terephthalate and/or below the boiling point of monoethylene glycol, in particular at 160°C.

7. Method according to any of the preceding claims, **characterised in that** inert gas, preferably nitrogen, is introduced into the reactor vessel.

8. Method according to any of the preceding claims, **characterised in that** alkylene glycol is removed from a reaction discharge, preferably by means of evaporation.

9. Method according to any of the preceding claims, **characterised in that** water is added to the reaction discharge to dissolve solid constituents, solids preferably being filtered out of the reaction discharge.

10. Method according to any of the preceding claims, **characterised in that** an acid is added to the reaction discharge in order to convert carboxylate ions contained in the reaction discharge and formed during the depolymerisation into acid.

11. Device for carrying out a method for reprocessing according to any of claims 1 to 10, comprising a reactor vessel which has conveying means, and means for supplying a preferably solid alkali hydroxide and/or alkaline earth hydroxide and means for supplying an alkylene glycol into the reactor vessel, wherein the reactor vessel is designed as a twin-screw extruder, preferably rotating in the same direction.

12. Device according to claim 11, **characterised in that** the extruder has at least one screw element, the outer diameter of which has a ratio to the inner diameter of approximately 1.7 and/or **in that** a length of the screw element has a ratio to the outer diameter of approximately 60.

13. Device according to either claim 11 or claim 12, **characterised in that** the conveying means have conveying, neutral and/or returning screw elements arranged one behind the other in order to convey, knead and/or return the reaction mixture, in portions, in the reactor.

14. Device according to claim 13, **characterised in that** the reactor vessel is provided with means for temperature control, in portions, that is adapted to the screw elements.

15. Use of a device according to any of claims 11 to 14 for carrying out a method according to any of claims 1 to 10.

## Revendications

1. Procédé de retraitement de déchets contenant sensiblement du polytéréphtalate d'alkylène, en particulier du polytéréphtalate d'éthylène et/ou de polytéréphtalate de butylène, en un processus continu au moyen d'une dépolymérisation, dans lequel un hydroxyde alcalin et/ou alcalino-terreux, en particulier de l'hydroxyde de sodium, est ajouté aux déchets pour la fabrication d'un mélange réactionnel, **caractérisé en ce qu'**en supplément un alkylène glycol est ajouté en tant que produit de départ au mélange réactionnel, dans lequel l'alkylène glycol est un alkylène glycol, en particulier monoéthylene glycol, pouvant être fabriqué en tant que produit de la dépolymérisation visée, et dans lequel aucun autre constituant réactif n'est ajouté au mélange réactionnel, dans lequel une extrudeuse à double vis sans fin est utilisée pour le refoulement, dans lequel de préférence les vis sans fin sont tournées dans le même sens.

2. Procédé selon la revendication 1, **caractérisé en ce que** les déchets sont broyés avant la fabrication du mélange réactionnel, de préférence à une taille de 3 mm au maximum.

3. Procédé selon la revendication 1 ou 2, **caractérisé en ce que** l'alkylène glycol est ajouté avec un flux massique qui est choisi de telle manière que le rapport de flux massique entre les déchets et l'alkylène glycol est d'au moins 3, en particulier d'environ 3,3.

4. Procédé selon l'une quelconque des revendications précédentes, **caractérisé en ce que** l'hydroxyde alcalin et/ou alcalino-terreux est ajouté avec un flux massique tel que le rapport entre l'hydroxyde alcalin et/ou alcalino-terreux et le polytéréphtalate d'éthylène est à peu près stœchiométrique, en particulier d'environ 2,4, par rapport à un motif récurrent constitutionnel.

5. Procédé selon l'une quelconque des revendications précédentes, **caractérisé en ce que** le mélange réactionnel pour la dépolymérisation est refoulé en continu par un contenant de réacteur, dans lequel de préférence le mélange réactionnel est malaxé et/ou mélangé et/ou refoulé et/ou refoulé en retour au cours de la dépolymérisation.

6. Procédé selon l'une quelconque des revendications précédentes, **caractérisé en ce que** la dépolymérisation est effectuée à une température inférieure au point d'ébullition du polytéréphtalate d'alkylène et/ou inférieure au point d'ébullition du monoéthylène glycol, en particulier à 160 °C.

7. Procédé selon l'une quelconque des revendications précédentes, **caractérisé en ce que** du gaz inerte, de préférence de l'azote, est introduit dans le contenant de réacteur.

8. Procédé selon l'une quelconque des revendications précédentes, **caractérisé en ce que** de l'alkylène glycol est supprimé de l'effluent réactionnel, de préférence par évaporation.

9. Procédé selon l'une quelconque des revendications précédentes, **caractérisé en ce que** de l'eau est ajoutée à l'effluent réactionnel pour dissoudre des constituants solides, dans lequel de préférence
des matières solides sont filtrées hors de l'effluent réactionnel.

10. Procédé selon l'une quelconque des revendications précédentes, **caractérisé en ce qu'**un acide est ajouté à l'effluent réactionnel pour transformer en acide des ions de carboxylate obtenus lors de la dépolymérisation, contenus dans l'effluent réactionnel.

11. Procédé pour réaliser un procédé de retraitement selon l'une quelconque des revendications 1 à 10, avec un contenant de réacteur, lequel présente des moyens de refoulement, ainsi qu'avec des moyens pour amener un hydroxyde alcalin et/ou alcalino-terreux de préférence solide et des moyens pour amener un alkylène glycol dans le contenant de réacteur, dans lequel
le contenant de réacteur est formé en tant qu'extrudeuse à double vis sans fin, les vis sans fin tournant de préférence dans le même sens.

12. Dispositif selon la revendication 11, **caractérisé en ce que** l'extrudeuse présente au moins un élément de vis, dont le diamètre extérieur par rapport au diamètre intérieur se trouve dans un rapport d'environ 1,7, et/ou
qu'une longueur par rapport au diamètre extérieur se situe dans un rapport d'environ 60 dans le cas d'un élément de vis sans fin.

13. Dispositif selon l'une quelconque des revendications 11 à 12, **caractérisé en ce que** les moyens de refoulement présentent des éléments de vis sans fin de refoulement, de refoulement neutre et/ou de refoulement en retour disposés les uns derrière les autres pour refouler, malaxer et/ou refouler en retour par endroits le mélange réactionnel dans le réacteur.

14. Dispositif selon la revendication 13, **caractérisé en ce que** le contenant de réacteur est pourvu de moyens de thermorégulation par endroits adaptés aux éléments de vis sans fin.

15. Utilisation d'un dispositif selon l'une quelconque des revendications 11 à 14 pour réaliser un procédé selon l'une quelconque des revendications 1 à 10.
